# EUROPEAN PATENT APPLICATION

(11) **EP 4 137 506 A1**
(43) Date of publication of application: **22.02.2023**
(21) Application number: 21788370.1
(22) Date of filing: 14.04.2021
(51) Int. Cl.: C07K 14/005, A61K 39/187, A61P 31/20, A61K 39/00

(54) **AFRICAN SWINE FEVER VACCINE COMPOSITION**

(30) Priority: 14.04.2020 KR 20200045271
(71) Applicant: Plumbline Life Sciences, Inc., Seoul 06661 (KR)
(72) Inventor: KIM, Eun Jin, Goyang-si Gyeonggi-do 10371 (KR); UHM, Tae-Gi, Yongin-si Gyeonggi-do 16944 (KR); SONG, Kyung-Joo, Seoul 05307 (KR); KIM, Min-Ji, Gangneung-si Gangwon-do 25419 (KR); YOUK, Geun-Young, Seoul 02147 (KR)
(74) Representative: Rückerl, Florian
(86) International application number: PCT/KR2021/004724
(87) International publication number: WO 2021/210924

(57) **Abstract**

The present invention relates to a polypeptide, a polynucleotide, a plasmid and a vaccine composition comprising the same, which are involved in eliciting immune responses to African Swine Fever. And also, it relates to a method of eliciting immune responses to African Swine Fever in a subject. In addition, it relates to a pharmaceutical composition for treating or preventing African Swine Fever comprising a polypeptide, a polynucleotide or a plasmid, which is involved in eliciting immune responses to African Swine Fever. Also, it relates to a method of treating or preventing African Swine Fever in a subject.

## Description

### [Technical Field]

The present invention relates to a polypeptide, a polynucleotide, a plasmid and a vaccine composition comprising the same, which are involved in eliciting immune responses to African Swine Fever. And also, it relates to a method of eliciting immune responses to African Swine Fever in a subject. In addition, it relates to a pharmaceutical composition for treating or preventing African Swine Fever comprising a polypeptide, a polynucleotide or a plasmid, which is involved in eliciting immune responses to African Swine Fever. Also, it relates to a method of treating or preventing African Swine Fever in a subject.

### [Background Art]

African Swine Fever (ASF) is a contagious viral disease of pigs that causes severe bleeding. Once occurred, it is highly contagious, and there is no treatment. In addition, infected pigs have a very high mortality rate, and thus it causes great damage to the pork industry. Therefore, when African Swine Fever occurs, it must be immediately reported to the World Organization for Animal Health (OIE), and international trade related to pigs is immediately stopped. ASF is classified as a very important disease by the World Organization for Animal Health (OIE), and it is designated and managed as a legal communicable disease under the act on the Prevention of Contagious Animal Diseases in Korea.

African Swine Fever Virus (ASFV) is a DNA virus of about 200 nm that belongs to the genus Asfivirus of the family Asfarviridae. It has been reported that the ASF virus does not infect humans or other animals, but only infects animals belonging to the family Suidae.

ASF virus is present in large quantities in saliva, respiratory secretions, urine, feces, etc. derived from infected animals, and ASF is infected to normal animals that come into contact with these materials. Since the virus may remain alive in the blood and tissues of pigs after death, ASF may become contagious rapidly if a feed containing tissue of infected animals is provided to pigs without heating. Otherwise, the virus may be transmitted by blood spilled during fights between pigs, bloody diarrhea, etc. Otherwise, blood-sucking insects such as mosquitoes, biting flies, and soft ticks belonging to *Ornithodoros spp.* may carry the ASFV and act as carriers that transmit the disease when they bite pigs or suck their blood.

ASF can infect pigs of all ages. The morbidity depends on the virus infected and the route of exposure, and the incubation period in natural infection varies from 4 to 19 days. Mortality is almost 100% when infected with a highly pathogenic virus.

ASF viruses can be classified into highly pathogenic, moderately pathogenic and low pathogenic depending on their pathogenicity. Highly pathogenic ASF viruses cause peracute (pig death 1 to 4 days after infection) and acute (pig death 3 to 8 days after infection) disease, and moderately pathogenic ASF viruses cause acute (pig death 11 to 15 days after infection) and subacute (pig death 20 days after infection) disease. Low pathogenic ASF viruses have been reported only in endemic areas, and cause subclinical or chronic disease.

ASF is difficult to diagnose at an early stage, especially when the number of infected pigs is small. This is because the clinical symptoms of ASF are confused with other hemorrhagic swine diseases such as septicaemic salmonellosis and porcine dermatitis nephropathy syndrome (PDNS). Accurate diagnosis is possible only through laboratory analysis such as a method to detect viruses in blood and internal organs and a method to detect antibodies in the serum of infected pigs. In many cases, when ASF is diagnosed, the disease is already widely transmitted. For example, the contagiousness of ASF is so high that ASF occurred in all administrative regions of China in less than nine months after ASF was first diagnosed in 2018 in China. In addition, in China, the largest pig farming country in the world, about 130 million pigs were dead or killed due to ASF in 2019, which means a decrease of about 1/3 from the 430 million pigs before the ASF outbreak.

ASF vaccine has been steadily researched since the 1960s. Various attempts have been made to date, such as live attenuated vaccines, inactivated vaccines, recombinant protein vaccines, and viral vector vaccines, but a vaccine with excellent effects that can be commercially applied has not been developed. A study has been recently published showing that even though neutralizing antibodies were formed by vaccination with an inactivated vaccine, there was no or negligible protective effect during actual challenge. It shows that the induction of cellular immune response, in addition to the production of neutralizing antibodies, is an important mechanism for improving the protective ability of vaccines against viruses (Takamatsu et al., Virus Res. 2013 Apr, 173(1):110-21).

In addition, one of the reasons to be difficult in developing an effective vaccine against ASF, which is fatal in pigs, is the large and complex nature of the ASF virus. While normal viruses have 10 to 12 proteins, ASF viruses have more than 150 proteins, and there are many different types of viruses (24 genotypes). Therefore, experts have predicted that commercialization of vaccines is difficult within at least the next 10 years.

Therefore, a vaccine or treatment against ASF is highly desired. In particular, it is important to prevent ASF stably and effectively in advance, and there is a very high unmet need for new vaccines for this purpose.

### [Technical Problem]

It is one purpose of the present invention to provide a polypeptide capable of eliciting immune responses to ASF, and a polynucleotide encoding the same.

It is another purpose of the present invention to provide a plasmid capable of eliciting immune responses to ASF.

It is another purpose of the present invention to provide a vaccine composition against ASF.

It is another purpose of the present invention to provide a method of eliciting immune responses to ASF in a subject.

It is another purpose of the present invention to provide a pharmaceutical composition for treating or preventing ASF.

It is another purpose of the present invention to provide a method of treating or preventing ASF in a subject.

### [Technical Solution]

In order to achieve the above purposes, one aspect of the present invention provides a polypeptide comprising at least one amino acid sequence selected among ASF virus genes p30, p54, C-type lectin, CD2v, p49, pp62, EP364R, F317L, A104R and K205R.

Another aspect of the present invention provides a polypeptide comprising at least one selected among amino acid sequences of SEQ ID NOs: 1 to 10 or amino acid sequences having 90% or more identity thereto.

As used herein, the term "identity" refers to the degree to which a given polypeptide sequence or polynucleotide sequence corresponds, and may be expressed in percentage. In the present specification, the identity of a sequence having identical or similar activity to a given polypeptide sequence may be indicated by "% identity". The identity may be determined by using standard software such as BLAST 2.0, which computes parameters such as score, identity, similarity, etc., or by comparing sequences through a hybridization test used under strictly defined conditions. The appropriate hybridization conditions to be defined may be decided by any method well known to a person skilled in the art.

The amino acid sequences of SEQ ID NOs: 1 to 10 are, in order, respectively, are consensus sequences derived by comparing the sequence homology of genes p30 (CP204L), p54 (E183L), C-type lectin (EP153R), CD2v (EP402R), p49 (B438L), pp62 (CP530R), EP364R, F317L, A104R, and K205R from various strains of ASF virus.

A polypeptide comprising at least one among the amino acid sequences of SEQ ID NOs: 1 to 10 or amino acid sequences having 90% or more identity thereto can effectively act as an antigen capable of generating an immune response against ASF.

The polypeptide may comprise one of the above amino acid sequences, or may comprise two or more. In addition, the polypeptide may further comprise a Kozak sequence, an IgE leader sequence, a ubiquitin sequence, and/or a cleavage site sequence. For example, the polypeptide may comprise Kozak sequence - IgE leader sequence - amino acid sequence of SEQ ID NO: 1. As another example, the polypeptide may comprise Kozak sequence - IgE leader sequence - ubiquitin sequence - amino acid sequence of SEQ ID NO: 1. As another example, the polypeptide may comprise Kozak sequence - IgE leader sequence - ubiquitin sequence - amino acid sequence of SEQ ID NO: 1 - cleavage site sequence - amino acid sequence of SEQ ID NO: 2. The ubiquitin sequence may be preferably SEQ ID NO: 41, and the cleavage site sequence may be preferably SEQ ID NO: 43.

One aspect of the present invention provides a polynucleotide comprising at least one nucleotide sequence selected among ASF virus genes p30, p54, C-type lectin, CD2v, p49, pp62, EP364R, F317L, A104R and K205R.

Another aspect of the present invention provides a polynucleotide comprising at least one selected among nucleotide sequences of SEQ ID NOs: 11 to 20 or nucleotide sequences having 90% or more identity thereto.

Alternatively, another aspect of the present invention provides a polynucleotide encoding at least one among amino acid sequences of SEQ ID NOs: 1 to 10 or amino acid sequences having 90% or more identity thereto. The polynucleotides encoding the amino acid sequences of SEQ ID NOs: 1 to 10 may be nucleotide sequences of SEQ ID NOs: 11 to 20, respectively.

The nucleotide sequences of SEQ ID NOs: 11 to 20 are, in order, respectively, are consensus sequences derived by comparing the sequence homology of genes p30 (CP204L), p54 (E183L), C-type lectin (EP153R), CD2v (EP402R), p49 (B438L), pp62 (CP530R), EP364R, F317L, A104R, and K205R from various strains of ASF virus.

Alternatively, in the polynucleotide, the amino acid sequences of SEQ ID NOs: 1 to 10 or the nucleotide sequences of SEQ ID NOs: 11 to 20 may be those modified by codons optimized for a subject to generate an immune response. The subject is, for example, an animal. The animal may be a mammal including, for example, pig, cow, horse, sheep, goat, deer and human. Preferably, the animal is a pig.

The polynucleotide may comprise one of the above nucleotide sequences, or may comprise two or more. In addition, the polynucleotide may further comprise a Kozak sequence, an IgE leader sequence, a ubiquitin sequence, and/or a cleavage site sequence. For example, the polynucleotide may comprise Kozak sequence - IgE leader sequence - nucleotide sequence of SEQ ID NO: 11. As another example, the polynucleotide may comprise Kozak sequence - IgE leader sequence - ubiquitin sequence - nucleotide sequence of SEQ ID NO: 11. As another example, the polynucleotide may comprise Kozak sequence - IgE leader sequence - ubiquitin sequence - nucleotide sequence of SEQ ID NO: 11 - cleavage site sequence - nucleotide sequence of SEQ ID NO: 12. The ubiquitin sequence may be preferably SEQ ID NO: 42, and the cleavage site sequence may be preferably SEQ ID NO: 44.

One aspect of the present invention provides a plasmid comprising a polynucleotide comprising at least one nucleotide sequence selected among ASF virus genes p30, p54, C-type lectin, CD2v, p49, pp62, EP364R, F317L, A104R and K205R.

Another aspect of the present invention provides a plasmid comprising a polynucleotide comprising at least one selected among nucleotide sequences of SEQ ID NOs: 11 to 20 or nucleotide sequences having 90% or more identity thereto.

Another aspect of the present invention provides a plasmid comprising a polynucleotide encoding at least one among amino acid sequences of SEQ ID NOs: 1 to 10 or amino acid sequences having 90% or more identity thereto.

As used herein, the term "plasmid" refers to a DNA construct containing a DNA sequence operably linked to a suitable regulatory sequence capable of expressing DNA in an appropriate host or subject. The plasmid may be a vector, a phage particle, or simply a potential genome insert. In the present specification, the term plasmid may be interchangeably used with the term vector or virus vector.

The plasmid may be prepared according to a conventional method known in the art. For example, by inserting the polynucleotide into a plasmid vector through gene cloning, the plasmid according to the present invention may be prepared and used as a DNA vaccine.

The plasmid may comprise one of the above polynucleotide sequences, or may comprise two or more. In addition, the polynucleotide may further comprise a Kozak sequence, an IgE leader sequence, a ubiquitin sequence, and/or a cleavage site sequence.

For example, the plasmid may comprise the nucleotide sequence of SEQ ID NO: 31 in which the nucleotide sequence of SEQ ID NOs: 11 to 20 are sequentially linked. As another example, the plasmid may comprise the nucleotide sequence of SEQ ID NO: 36 in which a ubiquitin sequence and the nucleotide sequences of SEQ ID NOs: 11 to 20 are sequentially linked. For example, the plasmid may comprise the nucleotide sequence of SEQ ID NO: 50, wherein a ubiquitin sequence and the nucleotide sequences of SEQ ID NOs: 11 to 20 are sequentially linked, and also each of the nucleotide sequences of SEQ ID NOs: 11 to 20 is sequentially linked by a cleavage site sequence.

The ubiquitin sequence may preferably be the nucleotide sequence of SEQ ID NO: 42. CD8⁺ T cells recognize MHC class I-associated peptides derived from endogenous antigens such as viral antigens or oncogene products located in the cytosol. Prior to antigen presentation by these MHC class I molecules, antigens are ubiquitinated and processed with antigenic peptides by the proteasome. Therefore, ubiquitin-fused proteins enter the proteasome dependent degradation pathway and enhance the ability to present MHC class I peptides, thereby increasing the induction of cellular immune response (CTL, Cytotoxic T Lymphocyte response).

The cleavage site sequence may preferably be the nucleotide sequence of SEQ ID NO: 44. The cleavage site refers to a peptide that is recognized and cleaved by a protease. In the present invention, it allows the polypeptide expressed from the plasmid containing the polynucleotide to be cleaved by a lyase, so that each cleaved polypeptide can effectively act as an antigen capable of generating an immune response against ASF. The cleavage site may be cleaved by an endogenous enzyme present in a cell, for example, but not limited to, a purine protease.

As another example, the plasmid may comprise the nucleotide sequence of SEQ ID NO: 32, wherein SEQ ID NO: 32 indicates that the nucleotide sequence of SEQ ID NOs: 11 to 14 are sequentially linked. Alternatively, the plasmid may comprise the nucleotide sequence of SEQ ID NO: 37, wherein SEQ ID NO: 37 indicates that a ubiquitin sequence and the nucleotide sequences of SEQ ID NOs 11 to 14 are sequentially linked.

As another example, the plasmid may comprise the nucleotide sequence of SEQ ID NO: 33, wherein SEQ ID NO: 33 indicates that the nucleotide sequence of SEQ ID NOs: 15 to 20 are sequentially linked. Alternatively, the plasmid may comprise the nucleotide sequence of SEQ ID NO: 38, wherein SEQ ID NO: 38 indicates that a ubiquitin sequence and the nucleotide sequences of SEQ ID NOs 15 to 20 are sequentially linked.

As another example, the plasmid may comprise the nucleotide sequence of SEQ ID NO: 34, wherein SEQ ID NO: 34 indicates that the nucleotide sequence of SEQ ID NOs: 11, 12, 16, 13 and 14 are sequentially linked. Alternatively, the plasmid may comprise the nucleotide sequence of SEQ ID NO: 39, wherein SEQ ID NO: 39 indicates that a ubiquitin sequence and the nucleotide sequences of SEQ ID NOs 11, 12, 16, 13 and 14 are sequentially linked.

As another example, the plasmid may comprise the nucleotide sequence of SEQ ID NO: 35, wherein SEQ ID NO: 35 indicates that the nucleotide sequence of SEQ ID NOs: 15, 17, 18, 19 and 20 are sequentially linked. Alternatively, the plasmid may comprise the nucleotide sequence of SEQ ID NO: 40, wherein SEQ ID NO: 40 indicates that a ubiquitin sequence and the nucleotide sequences of SEQ ID NOs 15, 17, 18, 19 and 20 are sequentially linked.

As another example, the plasmid may comprise the nucleotide sequence of SEQ ID NO: 50, wherein SEQ ID NO: 35 indicates that it comprises the ubiquitin sequence of SEQ ID NO: 42 and the cleavage site sequence of SEQ ID NO: 44, and the nucleotide sequence of SEQ ID NOs: 11, 44, 12, 44, 13, 44, 14, 44, 15, 44, 16, 44, 17, 44, 18, 44, 19, 44, 20 are sequentially linked.

As another example, the plasmid may comprise the nucleotide sequence of SEQ ID NO: 51, wherein SEQ ID NO: 51 indicates that it comprises the ubiquitin sequence of SEQ ID NO: 42 and the cleavage site sequence of SEQ ID NO: 44, and the nucleotide sequence of SEQ ID NOs: 11, 44, 12, 44, 13, 44, 14 are sequentially linked.

As another example, the plasmid may comprise the nucleotide sequence of SEQ ID NO: 52, wherein SEQ ID NO: 52 indicates that it comprises the ubiquitin sequence of SEQ ID NO: 42 and the cleavage site sequence of SEQ ID NO: 44, and the nucleotide sequence of SEQ ID NOs: 15, 44, 16, 44, 17, 44, 18, 44, 19, 44, 20 are sequentially linked.

As another example, the plasmid may comprise the nucleotide sequence of SEQ ID NO: 53, wherein SEQ ID NO: 53 indicates that it comprises the ubiquitin sequence of SEQ ID NO: 42 and the cleavage site sequence of SEQ ID NO: 44, and the nucleotide sequence of SEQ ID NOs: 11, 44, 12, 44, 16, 44, 13, 44, 14 are sequentially linked.

As another example, the plasmid may comprise the nucleotide sequence of SEQ ID NO: 54, wherein SEQ ID NO: 54 indicates that it comprises the ubiquitin sequence of SEQ ID NO: 42 and the cleavage site sequence of SEQ ID NO: 44, and the nucleotide sequence of SEQ ID NOs: 15, 44, 17, 44, 18, 44, 19, 44, 20 are sequentially linked.

Another aspect of the present invention provides an ASF vaccine composition comprising the polypeptide, the polynucleotide or the plasmid according to the present invention.

Unless otherwise stated, the polypeptide, the polynucleotide, and the plasmid in the ASF vaccine composition are the same as those mentioned above.

The term "vaccine" refers to a biological agent containing an antigen immunizing a subject. That is, it refers to an immunogenic or antigenic substance administered to a human or animal, for example through injection or oral administration, so as to provide immunization to a subject for preventing a disease.

The vaccine may be a DNA vaccine. The term "DNA vaccine" refers to a vaccine that causes an immune response by artificially replicating some of the genes from pathogens, viruses, etc. and administering the same. These DNA vaccines have many advantages over existing protein vaccines, including advantages such that (i) the DNA vaccines can be synthesized only with genetic information of nature target pathogen antigens, so there is no need to handle the hazardous pathogen itself, (ii) only some of the genes required for inducing toxicity are used, so the DNA vaccine would not show any unexpected toxicity when administered into a subject, and (iii) due to the structural simplicity of plasmid DNA, it is possible to develop a vaccine by rapidly responding to various infectious diseases occurring suddenly.

The ASF vaccine composition may have protective immunity, preferably against ASF virus.

The vaccine composition of the present invention may comprise a veterinarily acceptable carrier. The term "veterinarily acceptable carrier" includes any and all solvents, dispersion media, coatings, immunoadjuvants, stabilizers, diluents, preservatives, antibacterial agents, antifungal agents, isotonic agents, adsorption delaying agents, etc. Examples of the carriers, excipients and diluents that may be comprised in the vaccine composition include lactose, dextrose, sucrose, sorbitol, mannitol, xylitol, maltitol, starch, glycerin, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil. Also, the vaccine composition of the present invention may be formulated in oral dosage forms such as powder, granules, tablets, capsules, suspensions, emulsions, syrups, aerosols, etc. or sterile injection solutions according to a conventional method. When formulating the vaccine composition, the composition may be prepared using any diluent or excipient such as generally used fillers, extenders, binders, wetting agents, disintergrants, surfactants, etc. Preferably, poly-L-Glutamic acid may be used to aid in stabilization.

The vaccine composition may be injected into a subject in various forms. "Injection" may be carried out by any one selected from the group consisting of subcutaneous injection, intramuscular injection, intradermal injection, intraperitoneal injection, nasal administration, intravenous injection, transdermal administration and oral administration.

The vaccine composition may comprise one or more adjuvants in order to improve or enhance immune responses. Suitable adjuvants include peptides, aluminum hydroxide, aluminum phosphate, aluminum oxide, mineral oil such as Marcol 52, vegetable oil, emulsifiers, or surface active substances such as lysolecithin, polycations, polyanions and the like.

Another aspect of the present invention provides a method of eliciting immune responses to ASF virus in a subject, comprising: administering to a subject the vaccine composition of the present invention.

Unless otherwise stated, the vaccine composition in the method of eliciting immune responses has the same meaning as that mentioned above.

The term "immune response" refers to the activation of the immune system of a host in response to the introduction of an antigen. The immune response may be in the form of a cellular immunity response, a humoral immunity response, or both of them.

The term "subject" refers to animals including human, and may be, for example, mammals, particularly pig, cow, horse, sheep, goat, deer, human, and the like. It may be interchangeably used with an individual or a host.

In the method of eliciting immune responses, the vaccine composition may comprise an effective amount of active ingredients, i.e., a recombinant polypeptide, a recombinant polynucleotide or a plasmid comprising the same, with a pharmaceutically acceptable carrier and/or adjuvant. The term "effective amount" means that the ingredients of the vaccine are in an amount sufficient to induce an immune response specific to the ASF virus in the vaccinated animal. The effective amount may be easily determined by a person skilled in the art, for example through typical experiments on animals.

The administration may be carried out by any route suitable for administering the DNA vaccine, and may be carried out, for example, by subcutaneous, intramuscular, intraperitoneal, or intravenous injection.

Another aspect of the present invention provides a method for treating or preventing ASF in a subject, comprising administering to the subject the vaccine composition, polypeptide, polynucleotide or plasmid of the present invention.

Another aspect of the present invention provides a pharmaceutical composition for treating or preventing ASF, comprising the polypeptide, polynucleotide or plasmid according to the present invention.

In the method for treating or preventing ASF and the pharmaceutical composition for treating or preventing ASF according to the present invention, each term has the same meaning as described above unless otherwise stated.

The term "prevention" or "preventing" refers to any action in which ASF is suppressed or delayed by administration of the composition according to the present invention. In addition, the term "treatment" or "treating" refers to any action that improves, cures, improves, or partially treats symptoms of ASF by administration of the composition according to the present invention.

The vaccine composition according to the present invention may provide a DNA vaccine showing an excellent protective immunity against ASF virus. In particular, it has a very good effect of inducing a cellular immune response against ASF.

When the vaccine composition of the present invention is administered, a remarkably high level of antibody against ASF is produced in the inoculated subject, and accordingly an immune response is effectively exhibited and an excellent preventive effect against ASF is achieved.

### [Brief Description of Figures]

FIG. 1 is a cleavage map of a vector comprising an African Swine Fever (ASF) virus gene, which comprises a Kozak sequence, an IgE leader sequence, a ubiquitin sequence, and a furin cleavage site sequence, and comprises p30, p54, C-type lectin, CD2v, p49, pp62, EP364R, F317L, A104R and K205R as ASF virus genes.
FIG. 2 is a cleavage map of a vector comprising an ASF virus gene, which comprises a Kozak sequence, an IgE leader sequence, a ubiquitin sequence, and a furin cleavage site sequence, and comprises p30, p54, C-type lectin and CD2v as ASF virus genes.
FIG. 3 is a cleavage map of a vector comprising an ASF virus gene, which comprises a Kozak sequence, an IgE leader sequence, a ubiquitin sequence, and a furin cleavage site sequence, and comprises p49, pp62, EP364R, F317L, A104R and K205R as ASF virus genes.
FIG. 4 is a cleavage map of a vector comprising an ASF virus gene, which comprises a Kozak sequence, an IgE leader sequence, a ubiquitin sequence, and a furin cleavage site sequence, and comprises p30, p54, pp62, C-type lectin and CD2v as ASF virus genes.
FIG. 5 is a cleavage map of a vector comprising an ASF virus gene, which comprises a Kozak sequence, an IgE leader sequence, a ubiquitin sequence, and a furin cleavage site sequence, and comprises p49, EP364R, F317L, A104R and K205R as ASF virus genes.

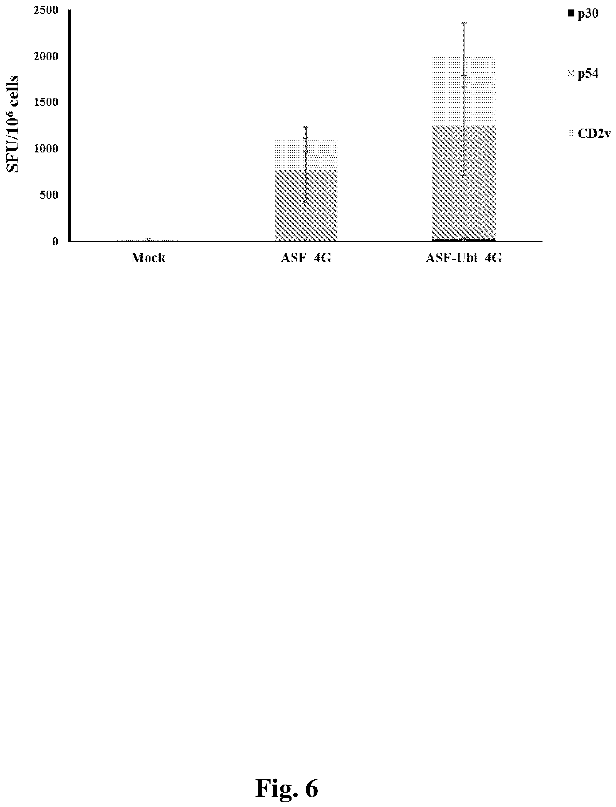

FIG. 6 is a graph showing the number of cytotoxic T cells (CD8⁺ T cells) generated by ASF virus antigens (p30, p54 or CD2v) after inoculation with a DNA vaccine comprising ASF virus genes. It shows the level of cellular immune response induced by the DNA vaccine. 'Mock' indicates the Control group administered with a DNA vaccine that does not comprise ASF virus genes. 'ASF_4G' indicates the Experimental group 1 administered with a DNA vaccine that comprises ASF virus genes (p30, p54, C type lectin and CD2v) and does not comprise ubiquitin, and 'ASF_Ubi_4G' indicates the Experimental group 2 administered with a DNA vaccine comprising ASF virus genes (p30, p54, C type lectin and CD2v) and ubiquitin.
FIG. 7 is a graph showing the survival rate of ASFV-infected pigs after inoculation with a DNA vaccine comprising ASF virus genes. 'Mock' indicates the Control group administered with a DNA vaccine that does not comprise ASF virus genes, and 'ASF_Ubi_10G' indicates the Experimental group administered with a DNA vaccine comprising ASF virus genes (p30, p54, C-type lectin, CD2v, p49, pp62, EP364R, F317L, A104R and K205R) and ubiquitin.

### [EXAMPLES]

Hereinafter, the present invention is described in more detail with examples. However, the examples are only for illustrating the present invention, and the scope of the present invention is not limited thereto.

### Example 1. ASF virus genes

The amino acid sequence of SEQ ID NO: 1 and the nucleotide sequence of SEQ ID NO: 11 are consensus sequences derived by comparing the sequence homology of the p30 (CP204L) gene from various strains of ASF virus. Specifically, the amino acid sequence of SEQ ID NO: 1 and the nucleotide sequence of SEQ ID NO: 11 were prepared by comparing sequence homology of the p30 (CP204L) gene from ASF virus strains ANG/70 (GenBank accession no. EU874271), Malawi/1978 (GenBank accession no. JQ744998), NAM/1/80 (GenBank accession no. JQ745005), Dedza (GenBank accession no. JQ745028), BUR/90/1 (GenBank accession no. EU874299), MOZ/94/1 (GenBank accession no. EU874263), DED 91/1 (GenBank accession no. KC867513), GUL 88/1 (GenBank accession no. KC867514), KAC 91/2 (GenBank accession no. KF736439), KANA 89/1 (GenBank accession no. KF736440), Killean I (GenBank accession no. JQ764860), Killean II (GenBank accession no. KC867521), Killean III (GenBank accession no. JQ764861), Kimakia I (GenBank accession no. JQ764954), Kimakia II (GenBank accession no. KC867515), KIRT 89/2 (GenBank accession no. JQ764858), KIRT 89/3 (GenBank accession no. JQ764856), KIRT 89/4 (GenBank accession no. JQ764857), KLI 88/2 (GenBank accession no. KC867516), LIL 89/1 (GenBank accession no. KC867517), LIL 90/1 (GenBank accession no. KF736437), LIV 5/40 (GenBank accession no. KC867518), LIV 9/31 (GenBank accession no. JQ764966), LIV 9/35 (GenBank accession no. JQ764965), LIV 10/11 (GenBank accession no. KC867519), LIV 12/17 (GenBank accession no. JQ764967), Mchinji 075 (GenBank accession no. JQ764880), MOZ 2001/1 (GenBank accession no. KC867524), MPO 89/1 (GenBank accession no. KC867520), NYA1/2 (GenBank accession no. EU874302), SAL 92/1 (GenBank accession no. KF736441), TEN 89/1 (GenBank accession no. KF736442), THY 90/1 (GenBank accession no. KF736438), Trench (GenBank accession no. JQ764859), MOZ/1960 (GenBank accession no. EU874309), Lillie (GenBank accession no. EU874306), 24823 (GenBank accession no. KC867500), MOZ/1979 (GenBank accession no. EU874310), SPEC/154 (GenBank accession no. EU874291), SPEC/205 (GenBank accession no. EU874305), SPEC/209 (GenBank accession no. EU874290), SPEC/257 (GenBank accession no. EU874265), MOZ/94/8 (GenBank accession no. EU874276), E70 (GenBank accession no. AF462272), ZAM/2017/Mbala/1 (GenBank accession no. LC322014), CN201801 (GenBank accession no. MH735141), DB/HLJ/2018 (GenBank accession no. MK333184), M-78 (GenBank accession no. MK211505), BA71 (GenBank accession no. KP055815), BA71V (GenBank accession no. U18466), E75 (GenBank accession no. FN557520), OURT 88/3 (GenBank accession no. AM712240), Georgia 2007 (GenBank accession no. FR682468), Ken06.Bus (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/R25/2015 (GenBank accession no. MH025918), Uganda/R8/2015 (GenBank accession no. MH025916), Pol16_20186_o7 (GenBank accession no. MG939583), Pol17_03029_C201 (GenBank accession no. MG939587), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894), Ken05/Tk1 (GenBank accession no. KM111294), Ken05/Tk6 (GenBank accession no. HM745363), Ken05.DPk2 (GenBank accession no. HM745368), Ken08WH/4 (GenBank accession no. HM745390), Ken08Tk.2/1 (GenBank accession no. HM745380), Ken09Tk.13/1 (GenBank accession no. HM745382), Ken09Tk.19/2 (GenBank accession no. HM745386), Ken09Tk.19/11 (GenBank accession no. HM745388).

The amino acid sequence of SEQ ID NO: 2 and the nucleotide sequence of SEQ ID NO: 12 are consensus sequences derived by comparing the sequence homology of the p54 (E183L) gene from various strains of ASF virus. Specifically, the amino acid sequence of SEQ ID NO: 2 and the nucleotide sequence of SEQ ID NO: 12 were prepared by comparing sequence homology of the p54 (E183L) gene from ASF virus strains Tengani/60 (GenBank accession no. KF015886), ANG/70 (GenBank accession no. EU874327), BA71 (GenBank accession no. KP055815), Malawi/1978 (GenBank accession no. KC662380), Brazil/79 (GenBank accession no. KC535549), DomRep/79 (GenBank accession no. FJ238534), KAV/89/1 (GenBank accession no. KF015902), BUR/90/1 (GenBank accession no. EU874363), MOZ/94/1 (GenBank accession no. EU874342), CHK 89/2 (GenBank accession no. KF015921), KAC 91/2 (GenBank accession no. KF736421), KANA 89/1 (GenBank accession no. KF736422), Killean III (GenBank accession no. KF736423), Kimakia I (GenBank accession no. KF015924), KIRT 89/4 (GenBank accession no. KF736414), LIL 90/1 (GenBank accession no. KF736416), LIV 9/31 (GenBank accession no. KF015928), MAN 89/2 (GenBank accession no. KF015940), MOZ 2001/1 (GenBank accession no. KF736428), MPO 89/1 (GenBank accession no. KF736418), Trench (GenBank accession no. KF736420), MOZ/1960 (GenBank accession no. EU874371), Lillie (GenBank accession no. EU874341), Madagascar (GenBank accession no. KC662387), ZIM/92/1 (GenBank accession no. EU874345), SPEC/205 (GenBank accession no. EU874329), Co62 (GenBank accession no. FJ174387), E70 (GenBank accession no. FJ174389), Ba71V (GenBank accession no. U18466), E75 (GenBank accession no. FN557520), Hu90 (GenBank accession no. FJ174399), SS81 (GenBank accession no. FJ174403), Ori90 (GenBank accession no. FJ174407), Nu98.8B (GenBank accession no. FJ174418), OURT 88/3 (GenBank accession no. AM712240), Almodovar 99 (GenBank accession no. DQ028315), Almodovar 99/NE1 (GenBank accession no. DQ028317), Georgia 2007 (GenBank accession no. FR682468), Angola (GenBank accession no. FJ174424), Nig01 (GenBank accession no. FJ174426), Ug03H.1 (GenBank accession no. FJ174431), Ug64 (GenBank accession no. FJ174430), Ken05.DPU1 (GenBank accession no. HM745354), Ken08WH/4 (GenBank accession no. HM745333), ken09Tk.20/5 (GenBank accession no. HM745344), Con09/PN003 (GenBank accession no. HQ645949), TAN/08/Mazimbu* (GenBank accession no. GQ410767), TAN/08/Mabibo* (GenBank accession no. GQ410768), Arm07 (GenBank accession no. JX857494), Az08D (GenBank accession no. JX857501), Oren08 (GenBank accession no. JX857498), Rostov09 (GenBank accession no. JX857504), Tver0312/Novo (GenBank accession no. KJ627190), Bel13/Grodno (GenBank accession no. KJ627192), LT14/1490 (GenBank accession no. KJ627193), ETH/1 (GenBank accession no. KT795366), ET13/1504 (GenBank accession no. KU291452), ETH/017 (GenBank accession no. KT795369), Ken06.Bus (GenBank accession no. KM111295), Ken07.Kia (GenBank accession no. FJ174437), CON09/Bzz020 (GenBank accession no. HQ645950), Ken10/KakFA1 (GenBank accession no. KC112568), Ug10.Kumi (GenBank accession no. KC990876), BUR/90/2 (GenBank accession no. KF015897), Ug12.Wakiso (GenBank accession no. KC990885), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/R7/2015 (GenBank accession no. MH025917), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894), CN201801 (GenBank accession no. MH735140), Estonia 2014 (GenBank accession no. LS478113), Nig6_JS10 (GenBank accession no. KT961344).

The amino acid sequence of SEQ ID NO: 3 and the nucleotide sequence of SEQ ID NO: 13 are consensus sequences derived by comparing the sequence homology of the C-type lectin (EP153R) gene from various strains of ASF virus. Specifically, the amino acid sequence of SEQ ID NO: 3 and the nucleotide sequence of SEQ ID NO: 13 were prepared by comparing sequence homology of the C-type lectin (EP153R) gene from ASF virus strains Katanga/63 (GenBank accession no. KM609340), Uganda (GenBank accession no. KM609361), BA71 (GenBank accession no. KP055815), Davis (GenBank accession no. KM609336), Ba71V (GenBank accession no. U18466), E75 (GenBank accession no. FN557520), NH/P68 (GenBank accession no. AF481875), Mafra 86 (GenBank accession no. DQ026269), Coimbra 87 (GenBank accession no. DQ026268), OURT 88/3 (GenBank accession no. AM712240), Portalegre 90 (GenBank accession no. DQ026270), Barrancos 93 (GenBank accession no. DQ026267), Almodovar 99 (GenBank accession no. DQ026265), Almodovar 99/NE1 (GenBank accession no. DQ026266), Georgia 2007 (GenBank accession no. FR682468), Ken05/Tk1 (GenBank accession no. KM111294), Ken06.Bus (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/R25/2015 (GenBank accession no. MH025918), Uganda/R8/2015 (GenBank accession no. MH025916), Pol16_20186_o7 (GenBank accession no. MG939583), Pol17_03029_C201 (GenBank accession no. MG939587), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894), UGA (GenBank accession no. AF017039), PR5 (GenBank accession no. AF017037), M1 (GenBank accession no. AF017034), K1 (GenBank accession no. AF017033), CR3 (GenBank accession no. AF017029), CR1 (GenBank accession no. AF017028), LC-PP (GenBank accession no. KM609345), Magadi (GenBank accession no. kM609348), Bartlett (GenBank accession no. KM609335), Volgograd_2012/wb (GenBank accession no. KM609363), Volgograd_2012/dom (GenBank accession no. KM609362), Tver_2012/wb (GenBank accession no. KM609360), Rhodesia (GenBank accession no. KM609354), TSP80 (GenBank accession no. KM609359), STP-1 (GenBank accession no. KM609355), O-77 (GenBank accession no. KM609350), F-32 (GenBank accession no. KM609337), MK-200 (GenBank accession no. KM609347), Spencer (GenBank accession no. KM609357), Silva-1 (GenBank accession no. kM609356), Ndjassi-77 (GenBank accession no. kM609349), KK-262 (GenBank accession no. KM609341), K-49 (GenBank accession no. KM609339), L-57 (GenBank accession no. KM609344), L-50 (GenBank accession no. KM609343).

The amino acid sequence of SEQ ID NO: 4 and the nucleotide sequence of SEQ ID NO: 14 are consensus sequences derived by comparing the sequence homology of the CD2v (EP402R) gene from various strains of ASF virus. Specifically, the amino acid sequence of SEQ ID NO: 4 and the nucleotide sequence of SEQ ID NO: 14 were prepared by comparing sequence homology of the CD2v (EP402R) gene from ASF virus strains China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894), Belgium 2018/1 (GenBank accession no. LR536725), Pol17_03029_C201 (GenBank accession no. MG939587), Pol16_20186_o7 (GenBank accession no. MG939583), Uganda/R8/2015 (GenBank accession no. MH025916), Uganda/N10/2015 (GenBank accession no. MH025919), Estonia 2014 (GenBank accession no. LS478113), Davis (1959_Kenya) (GenBank accession no. KM609336), Killean II (1959_Kenya) (GenBank accession no. KM609372), Kimakia II (1961_Kenya) (GenBank accession no. KM609374), Ba71V (1971_Spain) (GenBank accession no. U18466), E75 (1975_Spain) (GenBank accession no. FN557520), Ca78 (Italy_1978) (GenBank accession no. KT718663), Ori85 (Italy_1985) (GenBank accession no. KT718668), Nu91.5 (Italy_1991) (GenBank accession no. KT718673), NH/P68 (Portugal_1968) (GenBank accession no. AF481875), Mafra 86 (Portugal_1986) (GenBank accession no. DQ026269), Coimbra 87 (Portugal_1987) (GenBank accession no. DQ026268), Portalegre 90 (GenBank accession no. DQ026270), Barrancos 93 (GenBank accession no. DQ026267), Almodovar 99/NE1 (GenBank accession no. DQ026266), OURT 88/3 (GenBank accession no. AM712240), FR682468 (GenBank accession no. Georgia 2007), Ken05/Tk1 (GenBank accession no. KM111294), Ken06.Bus (GenBank accession no. KM111295), Italy/47/SS/2008 (GenBank accession no. KX354450), Portugal/NHV/1968 (GenBank accession no. KM262845), Italy/26544/OG10 (GenBank accession no. KM102979), Benin 97/1 (GenBank accession no. AM712239), Bartlett (GenBank accession no. KM609335), Zavidovo-2012 (GenBank accession no. KM609392), Tver_2012/wb (GenBank accession no. KM609360), Rhodesia (GenBank accession no. KM609354), Nanyuki (GenBank accession no. KM609382), Krasnodar_2012/dom (GenBank accession no. KM609342), TS-7/27-230 (GenBank accession no. KM609388), TSP80 (GenBank accession no. KM609359), K-49 (GenBank accession no. KM609339), KK-262 (GenBank accession no. KM609341), Spencer (GenBank accession no. KM609357), MK-200 (GenBank accession no. KM609347), 691/88 (GenBank accession no. KM609334), F-32 (GenBank accession no. KM609337), O-77 (GenBank accession no. KM609350), P-60 (GenBank accession no. KM609351), PPA (GenBank accession no. KM609352), STP-1 (GenBank accession no. KM609355), BA71 (GenBank accession no. KP055815), STP-1-79 (GenBank accession no. KM609384), Malta (GenBank accession no. KM609380), Yamba-74 (GenBank accession no. KM609391), TKF (GenBank accession no. KM609387), Kimuele-6 (GenBank accession no. KM609375), E-70 (GenBank accession no. KM609369), Cuba-71 (GenBank accession no. KM609366), CU-80 (GenBank accession no. KM609365), Brazil-80 (GenBank accession no. KM609364), MNI-82 (GenBank accession no. KM609378), K-73 (Le Bray) (GenBank accession no. KM609370), Madeira (GenBank accession no. KM609379), Diamang (GenBank accession no. KM609367), L-50 (GenBank accession no. KM609343), L-57 (GenBank accession no. KM609344), Kikasa-77 (GenBank accession no. KM609371), VL (GenBank accession no. KM609390).

The amino acid sequence of SEQ ID NO: 5 and the nucleotide sequence of SEQ ID NO: 15 are consensus sequences derived by comparing the sequence homology of the p49 (B438L) gene from various strains of ASF virus. Specifically, the amino acid sequence of SEQ ID NO: 5 and the nucleotide sequence of SEQ ID NO: 15 were prepared by comparing sequence homology of the p49 (B438L) gene from ASF virus strains BA71 (GenBank accession no. KP055815), Ba71V (GenBank accession no. U18466), E75 (GenBank accession no. FN557520), OURT 88/3 (GenBank accession no. AM712240), Georgia 2007 (GenBank accession no. FR682468), Ken05/Tk1 (GenBank accession no. KM111294), Ken06.BUS (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/N10/2015 (GenBank accession no. MH025919), Pol16_20186_o7 (GenBank accession no. MG939583), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894).

The amino acid sequence of SEQ ID NO: 6 and the nucleotide sequence of SEQ ID NO: 16 are consensus sequences derived by comparing the sequence homology of the pp62 (CP530R) gene from various strains of ASF virus. Specifically, the amino acid sequence of SEQ ID NO: 6 and the nucleotide sequence of SEQ ID NO: 16 were prepared by comparing sequence homology of the pp62 (CP530R) gene from ASF virus strains BA71 (GenBank accession no. KP055815), Ba71V (GenBank accession no. U18466), E75 (GenBank accession no. FN557520), OURT 88/3 (GenBank accession no. AM712240), Georgia 2007 (GenBank accession no. FR682468), Ken05/Tk1 (GenBank accession no. KM111294), Ken06.BUS (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/N10/2015 (GenBank accession no. MH025919), Pol16_20186_o7 (GenBank accession no. MG939583), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894), Krasnodar (2012) (GenBank accession no. KJ380911).

The amino acid sequence of SEQ ID NO: 7 and the nucleotide sequence of SEQ ID NO: 17 are consensus sequences derived by comparing the sequence homology of the EP364R gene from various strains of ASF virus. Specifically, the amino acid sequence of SEQ ID NO: 7 and the nucleotide sequence of SEQ ID NO: 17 were prepared by comparing sequence homology of the EP364R gene from ASF virus strains BA71 (GenBank accession no. KP055815), Ba71V (GenBank accession no. U18466), E75 (GenBank accession no. FN557520), OURT 88/3 (GenBank accession no. AM712240), Georgia 2007 (GenBank accession no. FR682468), Ken05/Tk1 (GenBank accession no. KM111294), Ken06.BUS (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/N10/2015 (GenBank accession no. MH025919), Pol16_20186_o7 (GenBank accession no. MG939583), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894).

The amino acid sequence of SEQ ID NO: 8 and the nucleotide sequence of SEQ ID NO: 18 are consensus sequences derived by comparing the sequence homology of the F317L gene from various strains of ASF virus. Specifically, the amino acid sequence of SEQ ID NO: 8 and the nucleotide sequence of SEQ ID NO: 18 were prepared by comparing sequence homology of the F317L gene from ASF virus strains BA71 (GenBank accession no. KP055815), Ba71V (GenBank accession no. U18466), E75 (GenBank accession no. FN557520), OURT 88/3 (GenBank accession no. AM712240), Georgia 2007 (GenBank accession no. FR682468), Ken05/Tk1 (GenBank accession no. KM111294), Ken06.BUS (GenBank accession no. KM111295), Estonia 2014 F317L (GenBank accession no. LS478113), Benin 97/1 F317L (GenBank accession no. AM712239), Italy/26544/OG10 F317L (GenBank accession no. KM102979), Portugal/NHV/1968 F317L (GenBank accession no. KM262845), Italy/47/SS/2008 F317L (GenBank accession no. KX354450), Uganda/R35/2015 F317L (GenBank accession no. MH025920), Uganda/N10/2015 (GenBank accession no. MH025919), Pol16_20186_o7 (GenBank accession no. MG939583), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894).

The amino acid sequence of SEQ ID NO: 9 and the nucleotide sequence of SEQ ID NO: 19 are consensus sequences derived by comparing the sequence homology of the A104R gene from various strains of ASF virus. Specifically, the amino acid sequence of SEQ ID NO: 9 and the nucleotide sequence of SEQ ID NO: 19 were prepared by comparing sequence homology of the A104R gene from ASF virus strains BA71 (GenBank accession no. KP055815), Ba71V (GenBank accession no. U18466), E75 (GenBank accession no. FN557520), OURT 88/3 (GenBank accession no. AM712240), Georgia 2007 (GenBank accession no. FR682468), Ken05/Tk1 (GenBank accession no. KM111294), Ken06.BUS (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/N10/2015 (GenBank accession no. MH025919), Pol16_20186_o7 (GenBank accession no. MG939583), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180), ASFV-SY18 (GenBank accession no. MH766894).

The amino acid sequence of SEQ ID NO: 10 and the nucleotide sequence of SEQ ID NO: 20 are consensus sequences derived by comparing the sequence homology of the K205R gene from various strains of ASF virus. Specifically, the amino acid sequence of SEQ ID NO: 10 and the nucleotide sequence of SEQ ID NO: 20 were prepared by comparing sequence homology of the K205R gene from ASF virus strains BA71 (GenBank accession no. KP055815), Ba71V (GenBank accession no. U18466), E75 (GenBank accession no. FN557520), OURT 88/3 (GenBank accession no. AM712240), Georgia 2007 (GenBank accession no. FR682468), Ken05/Tk1 (GenBank accession no. KM111294), Ken06.BUS (GenBank accession no. KM111295), Estonia 2014 (GenBank accession no. LS478113), Benin 97/1 (GenBank accession no. AM712239), Italy/26544/OG10 (GenBank accession no. KM102979), Portugal/NHV/1968 (GenBank accession no. KM262845), Italy/47/SS/2008 (GenBank accession no. KX354450), Uganda/R35/2015 (GenBank accession no. MH025920), Uganda/N10/2015 (GenBank accession no. MH025919), Pol16_20186_o7 (GenBank accession no. MG939583), Pol17_04461_C210 (GenBank accession no. MG939588), Belgium 2018/1 (GenBank accession no. LR536725), China/2018/AnhuiXCGQ (GenBank accession no. MK128995), DB/LN/2018 (GenBank accession no. MK333181), Pig/HLJ/2018 (GenBank accession no. MK333180)ASFV-SY18 (GenBank accession no. MH766894).

### Example 2. Preparation of ASF vaccines

A vaccine was prepared using the ASF virus genes of Example 1.

Specifically, a DNA plasmid comprising said genes, Kozak sequence, IgE leader sequence, ubiquitin sequence, and/or Furin cleavage site sequence was prepared by a general method known in the art. And then, *E. coli* having the DNA plasmid was inoculated into 2.5 L of LB medium and cultured at 37°C in oil-in-water (O/W). Plasmids were extracted from the cultured *E. coli* using the EndoFree Plasmid Giga kit (QIAGEN, Cat#12391), and the extracted plasmids were subsequently used for inoculation as a vaccine.

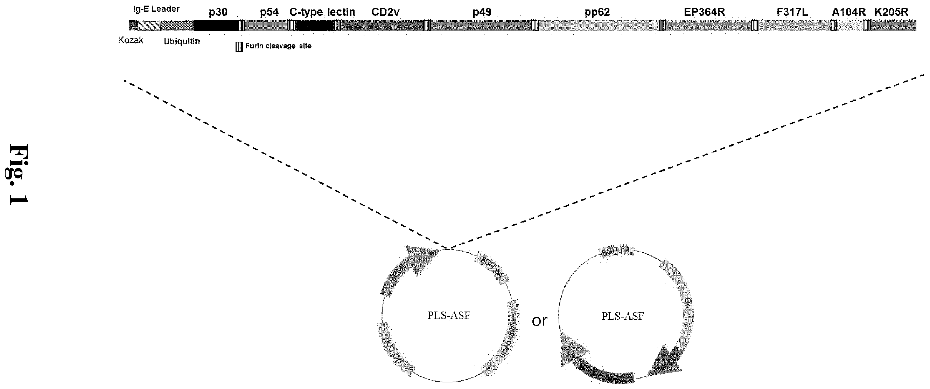

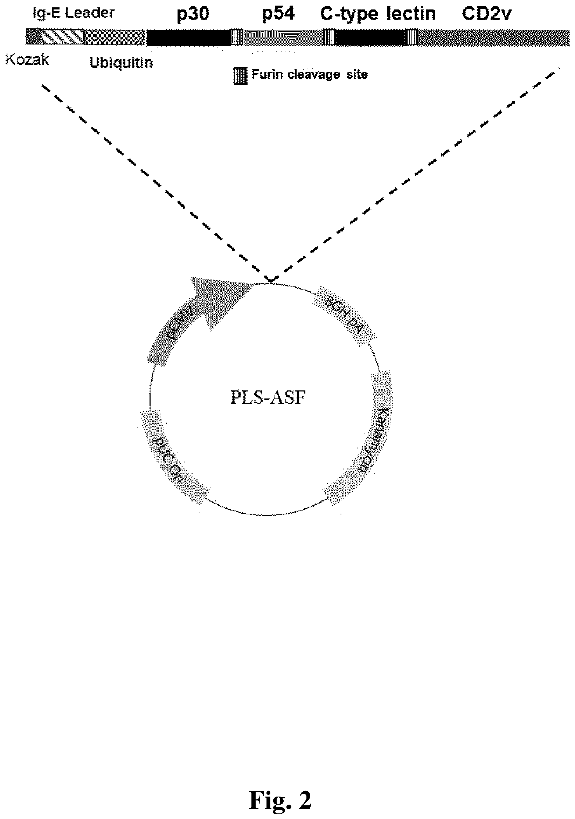

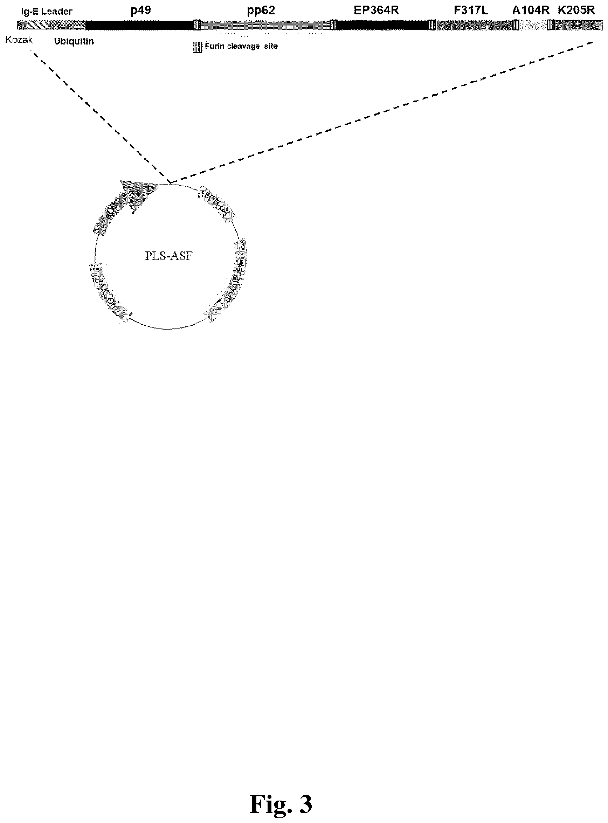

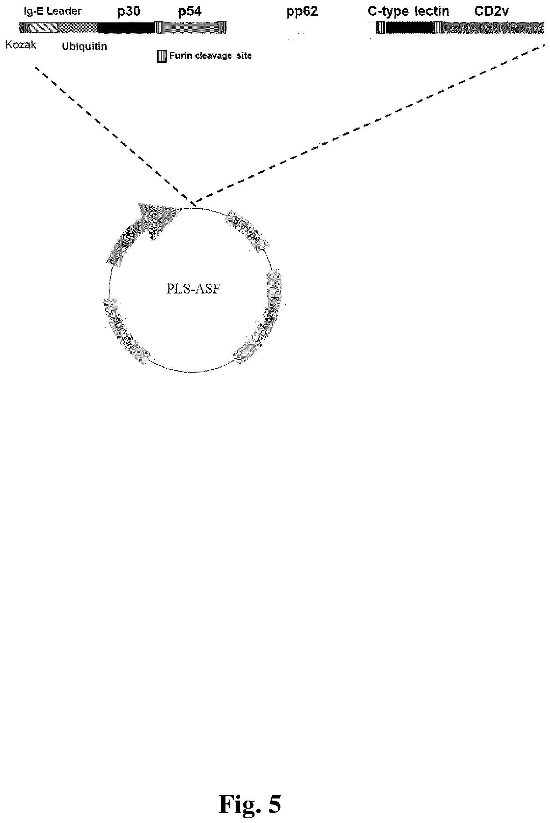

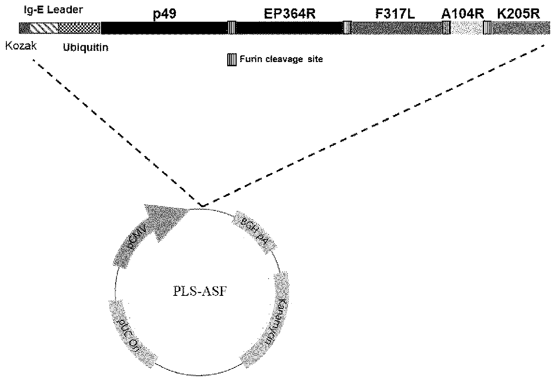

Cleavage maps of the prepared DNA plasmids are shown in FIGs. 1 to 5.

### Example 3. Cellular immune response induction effect of ASF vaccine

As the ASF preventive effect of the DNA vaccine prepared in Example 2, the cellular immune response (CTL, Cytotoxic T Lymphocyte response) inducing effect was evaluated.

Fifteen C57BL/6 mice (8-week-old, female) were divided into three groups of five mice per group, and each group was inoculated with the vaccine three times at intervals of two weeks. The DNA vaccine was administered at a concentration of 30 µg/mouse, and the administration and inoculation were carried out using an electroporation device (cellectra 2000). The type of DNA vaccine administered to each Control group and Experimental group was set as follows.
- Control group: DNA vaccine not comprising an ASF virus gene (Mock Plasmid DNA vaccine)
- Experimental group 1: DNA vaccine comprising ASF virus genes and not comprising ubiquitin (ASF_4G DNA vaccine; p30, p54, C type lectin and CD2v)
- Experimental group 2: DNA vaccine comprising ASF virus genes and ubiquitin (ASF_Ubi_4G DNA vaccine; ubiquitin, p30, p54, C type lectin and CD2v)

One week after DNA vaccine was administered three times, the spleen was removed from the mouse and splenocytes were isolated. Mouse IFN-gamma ELISpot analysis was performed using IFN-gamma ELISpot KIT (Cellular Technology Limited/USA) for the isolated splenocytes. Specifically, the splenocytes administered with each vaccine were treated with the polypeptide of each antigen gene, and then the number of cytotoxic T cells (CD8⁺ T cells) secreting IFN-γ was analyzed. Accordingly, the degree of cellular immune response induced by viral infection after vaccination was evaluated.

As a result, as shown in FIG. 6 and Table 1, it was confirmed that the number of cytotoxic T cells (CD8⁺ T cells) secreting IFN-γ was significantly increased when treated with the polypeptide of each antigen gene in Experimental groups 1 and 2 administered with the DNA vaccine containing the ASF virus gene.

**[Table 1]**

| | p30 | p54 | CD2v |
|---|---|---|---|
| Control group (Mock) | 0.0 | 0.0 | 8.5 |
| Experimental group 1 (ASF_4G) | 0.0 | 769.0 | 336.5 |
| Experimental group 2 (ASF_Ubi_4G) | 23 | 1223.5 | 764 |

| | | | |
|---|---|---|---|
| (Unit: SFU/10⁶ cells) | | | |

In particular, it was confirmed that the number of cytotoxic T cells (CD8⁺ T cells) secreting IFN-γ was increased by about 1.6 to 2.2 times in Experimental group 2 administering DNA vaccine comprising ubiquitin compared to Experimental group 1 administering DNA vaccine not comprising ubiquitin. In addition, among various antigen genes comprised in DNA vaccines, it was confirmed that the number of cytotoxic T cells (CD8⁺ T cells) secreting IFN-γ is particularly increased by p54 or CD2v, and that p30 increased the number of cytotoxic T cells (CD8⁺ T cells) secreting IFN-γ only when a vaccine containing ubiquitin is administered (Experimental group 2).

Through the above results, when vaccinated with a DNA vaccine containing the ASF virus genes and ubiquitin, the cellular immune response can be efficiently induced by remarkably increasing the number of cytotoxic T cells secreting IFN-γ even if infected with the ASF virus. Thus, it was confirmed that the DNA vaccine of the present invention can be usefully utilized as a vaccine composition for preventing ASF.

### Example 4. Effect of increasing survival rate by ASF vaccine

As the ASF preventive effect of the vaccine prepared in Example 2, the effect of increasing survival rate after ASF virus infection was evaluated.

Specifically, the DNA vaccine was administered to piglets three times (week 0, week 2, week 4). Thereafter, the ASF virus was challenged at 7 weeks, and then the survival rate for 28 days was examined. The type of DNA vaccine administered to each Control group and Experimental group was set as follows.
. Control group: DNA vaccine not comprising an ASF virus gene (Mock)
. Experimental group: DNA vaccine comprising ASF virus genes and ubiquitin (ASF_Ubi_10G DNA vaccine; ubiquitin, p30, p54, C-type lectin, CD2v, p49, pp62, EP364R, F317L, A104R and K205R)

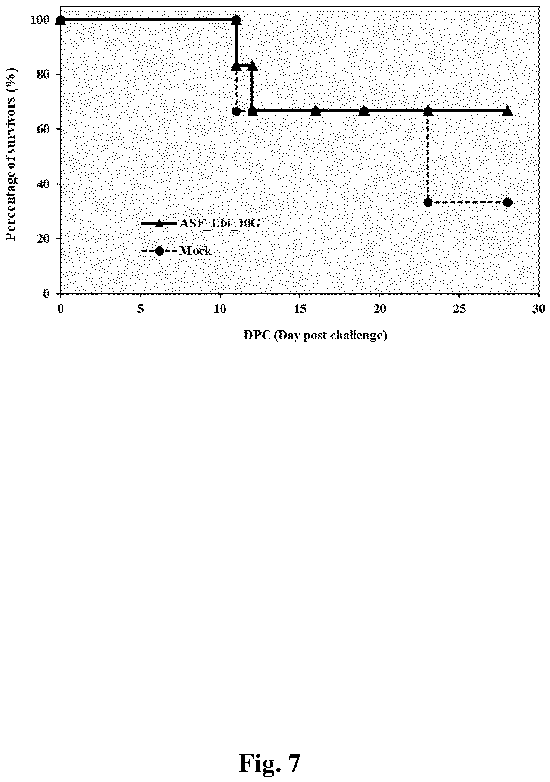

As a result, FIG. 7 shows that on the 28th day of inoculation in the Control group, only 1 out of 3 pigs survived, indicating a low survival rate of about 33%. On the contrary, on the 28th day of inoculation, in the Experimental group administered with the DNA vaccine containing the ASF virus gene, 4 out of 6 pigs survived, indicating a high survival rate of about 66%. That is, it was confirmed that the survival rate of the Experimental group increased more than twice compared to that of the Control group.

The above results show that when inoculated with a DNA vaccine comprising ASF virus gene and ubiquitin, the subject infected with ASF virus shows excellent survival ability, and thus the DNA vaccine of the present invention can be usefully used as a vaccine composition against ASF.

## Claims

1. A polypeptide comprising at least one amino acid sequence selected among African swine fever virus genes p30, p54, C-type lectin, CD2v, p49, pp62, EP364R, F317L, A104R and K205R.

2. The polypeptide of claim 1, wherein it comprises at least one selected among amino acid sequences of SEQ ID NOs: 1 to 10 or amino acid sequences having 90% or more identity thereto.

3. The polypeptide of claim 1, wherein it further comprises at least one selected among a Kozak sequence, an IgE leader sequence, a ubiquitin sequence and a furin cleavage site sequence.

4. The polypeptide of claim 1, wherein it further comprises an amino acid sequence of SEQ ID NO: 41.

5. The polypeptide of claim 1, wherein it further comprises an amino acid sequence of SEQ ID NO: 43.

6. A polynucleotide comprising at least one nucleotide sequence selected among African swine fever virus genes p30, p54, C-type lectin, CD2v, p49, pp62, EP364R, F317L, A104R and K205R.

7. The polynucleotide of claim 6, wherein it comprises nucleotide sequences encoding amino acid sequences of SEQ ID NOs: 1 to 10 or amino acid sequences having 90% or more identity thereto, or nucleotide sequences of SEQ ID NOs: 11 to 20 or nucleotide sequences having 90% or more identity thereto.

8. The polynucleotide of claim 6, wherein it further comprises at least one selected among a Kozak sequence, an IgE leader sequence, a ubiquitin sequence and a cleavage site sequence.

9. The polynucleotide of claim 6, wherein it further comprises a nucleotide sequence of SEQ ID NO: 42.

10. The polynucleotide of claim 6, wherein it further comprises a nucleotide sequence of SEQ ID NO: 44.

11. A plasmid comprising the polynucleotide of claim 6.

12. The plasmid of claim 11, wherein it comprises any one selected among nucleotide sequences of SEQ ID NOs: 31 to 40 and 50 to 54.

13. A vaccine composition against African swine fever, wherein it comprises the polypeptide of any one of claims 1 to 5, the polynucleotide of any one of claims 6 to 10, or the plasmid of any one of claims 11 and 12.

14. A method of eliciting immune responses to African swine fever virus in a non-human animal, comprising administering to the animal the vaccine composition of claim 13.

15. The method of claim 14, wherein the administration is performed by electroporation.

16. A method of treating or preventing African swine fever, comprising administering to a non-human animal the vaccine composition of claim 13.

17. The method of claim 16, wherein the administration is performed by electroporation.

18. A pharmaceutical composition for treating or preventing African swine fever, comprising the polypeptide of any one of claims 1 to 5, the polynucleotide of any one of claims 6 to 10, or the plasmid of any one of claims 11 and 12.
